Europäisches Patentamt

European Patent Office

Office européen des brevets

(19)

(11) Publication number: **0 343 449 B1**

## EUROPEAN PATENT SPECIFICATION

(12)

(45) Date of publication of patent specification: **10.03.93**

(51) Int. Cl.5: **G01N 33/00**, G01N 37/00, G01N 27/12

(21) Application number: **89108557.3**

(22) Date of filing: **12.05.89**

The file contains technical information submitted after the application was filed and not included in this specification

(54) **Method of and apparatus for determining moisture content in gases.**

(30) Priority: **21.05.88 GB 8812086**

(43) Date of publication of application:
**29.11.89 Bulletin 89/48**

(45) Publication of the grant of the patent:
**10.03.93 Bulletin 93/10**

(84) Designated Contracting States:
**DE FR GB IT**

(56) References cited:
**US-A- 3 894 419**
**US-A- 4 212 194**

(73) Proprietor: **Moisture Control & Measurement Limited**
**Thorp Arch Trading Estate**
**Wetherby West Yorkshire LS23 7BJ(GB)**

(72) Inventor: **Wallis, Bruce, c/o Moisture Control& Measurement**
**Thorp Arch Trading Estate**
**Wetherby, Leeds, LS23 7BJ(GB)**

(74) Representative: **Denmark, James**
**Bailey, Walsh & Co. 5 York Place**
**Leeds LS1 2SD Yorkshire (GB)**

Rank Xerox (UK) Business Services
(3.10/3.5x/3.0.1)

EP 0 343 449 B1

## Description

The invention relates to a method of and apparatus for determining moisture content in gases particularly when the concentration is below one part per million.

Particularly in the semi-conductor field there is an ever increasing demand for the purity of gases and in particular for extremely low level of moisture content. At the same time, moisture-sensitive devices have become available which are extremely sensitive, but standardisation techniques have not yet enabled them to be calibrated at very low levels for example one part per million or less, and extrapolation below that level does not yield reliable results. Moreover, at very low levels for example one part per million or less even the most sensitive hygrometers take a long time to come to equilibrium which problem is compounded if the moisture content is likely to vary during measurement. These deficiencies have in practical terms prevented the full exploitation of the ultra-sensitive hygrometer.

According to one aspect of the invention there is provided a method for the determination of the moisture content of a gas stream comprising steps of:-

(a) adding a first quantity of moisture to the stream and determining the first combined water content;

(b) adding to the stream a second quantity of water having a determined relationship with the said first added quantity, and determining the second combined moisture content; and

(c) combining the results in (a) and (b) above to eliminate the contribution of the added moisture.

Preferably the second quantity of moisture bears a simple relation to the first added quantity. Preferably the added quantities are in the range above one part per million.

According to a further aspect of the invention there is provided apparatus for the determination of moisture content in a gas stream comprising a conduit for containing the gas stream, a hygrometer means located in the stream, and means including an auxiliary passage means for introducing first and second quantities of added moisture to the stream which means is located upstream of the hygrometer means. The moisture augmentation means may comprise a diffusion generator, and may further comprise means for providing that a known fraction of the augmented stream shall bypass the hygrometer means.

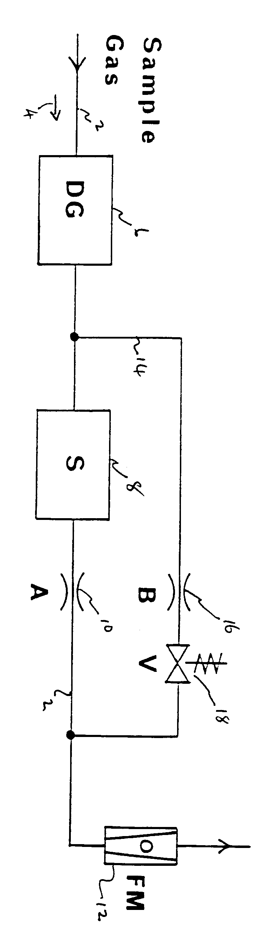

Embodiments of the invention will now be described by way of example with reference to the accompanying drawings, of which the single figure shows, schematically, apparatus for the measurement of the moisture content in a gas sample.

The gas of which the moisture content is to be measured is passed along conduit 2 in the direction of arrow 4. Typically, the gas may be a nominally dry and pure gas for example for use in the semi-conductor industry. Alternatively the gas may derive from a stream of dry gas passing through an apparatus in order to check whether there are leaks into the apparatus such as would allow ambient moisture to enter into the apparatus and thus contaminate the stream.

The conduit 2 leads to a diffusion generator 6 which will be referred to below and thence successively to a hygrometer 8 and the first 10 of two flow orifices before reaching a flow meter 12. The hygrometer is a silicon based hygrometer calibrated in the range 1-5 parts per million having a discrimination in the parts per billion level and having an accuracy of + 0.1 ppm at 1 ppm and + 0.35 ppm at 5 ppm level.

Between the diffusion generator 6 and the hygrometer 8 the conduit branches into an auxiliary passage 14. In this line are the other flow orifice 16 and a solenoid valve 18 beyond which the branch conduit re-unites with the main conduit between the first mentioned flow orifice and the flow meter 12.

Conveniently, all the components referred to except the flow meter, that is to say the diffusion generator, the hygrometer, the flow orifices, valve and main and branch conduits, form a single assembly.

In use of the apparatus valve 18 is closed so that gas is able to pass only along the main conduit 2, and the rate of flow given constant pressure, is controlled by the orifice 10. The flow rate is monitored by the flow meter 12. As the gas flows at this rate through the diffusion generator 6, the diffusion generator introduces a known amount of moisture (2K) into the gas stream. Whilst the sample gas may have moisture content well below one part per million the quantity of moisture added by the diffusion generator is arranged to be in the range 2 to 4 ppm, which ensures that the total moisture passing through the hygrometer 8 is within the range of accurate calibration of that hygrometer. Moreover, at that level of moisture content, equilibrium conditions are established within a few minutes rather than the hours taken to reach equilibrium with a moisture content in the parts per billion range.

When the equilibrium value R1 as given by the hygrometer has been recorded, the valve 18 is opened so that the sample gas is able to pass along the main and branch conduits and specifically through the orifices 10 and 16 in parallel. As the orifices are at least of approximately equal size, the total flow, which can be checked by the flow meter 12, is double that of the first test. It is a known property of a diffusion generator that it yields the same quantity of moisture in unit time irrespective of the rate of flow of the medium into which it is dispersed, so that effectively the moisture added per unit volume of the sample gas is half the previously added value. The flow of gas through the hygrometer 8 remains as before, controlled by the orifice 10, so that the scale reading R2, which is still largely dependent on the added moisture rather than the original moisture, is still in the range of reliable calibration and is still achieved after a relatively short equilibrium period.

It will be understood that the first moisture reading is essentially composed of two parts, namely W (the moisture content of the sample gas) and 2K (the moisture introduced by the diffusion generator in the first test), such that

$$R1 = W + 2K$$

Similarly, the moisture resulting in the second reading R2 is composed of two parts namely W (the moisture content of the sample gas) and K (the moisture introduced by the generator in the second test) so that

$$R2 = W + K$$

The above equasions can be solved in the conventional manner, eliminating K in the process and defining W, the moisture content of the sample gas, in terms of the difference between the two readings, that is to say:

$$W = 2R2 - R1$$

If preferred, the reading with the valve 18 open may be made before that with the valve closed.

It will be appreciated from the above calculation that the absolute levels of K do not matter. Thus, troublesome parameters which affect high sensitive determinations such as precise temperature control, control of absolute pressure etc., are not critical, so long as they are stable for the relatively short period of the tests. Even the very difficult requirement of accurate flow measurement is avoided since all that it need be guaranteed is that the flow ratio in the respective orifices is constant. The method assumes that for the short duration of the test the moisture content of the sample gas remains substantially constant, and this assumption is more assuredly made in view of the relatively fast response of the apparatus.

If the method is used for testing the purging of apparatus in which the moisture control is expected to diminish with time, the operation is modified to incorporate two R1 readings which are averaged prior to deriving W. In these circumstances it is clearly of advantage that the duration of the test is relatively short.

The influence of moisture leakage rates through fittings or outgassing of moisture from the pipe work is eliminated since each one can be considered at individual miniature diffusion generators, introducing moisture at a rate which is constant in unit time irrespective of flow rate at least in the 2:1 ratio of the tests.

It is not essential that the orifices shall pass exactly equal flows, provided that the ratio between them is known or can be measured and the calculations to find W is amended accordingly.

As an alternative to a diffusion generator, any other means of introducing a fixed quantity of water in unit time could be used.

## Claims

1. A method for the determination of the moisture content of a gas stream where the moisture content may be less than 1 ppm and where the moisture content may also be likely to vary during measurement characterized in that:

   (a) a first quantity of moisture is added to the stream and the first combined moisture content is determined;

   (b) a second quantity of moisture having a determined relationship with the said first quantity is added to the stream and the second combined moisture content is determined;

   (c) the results of the said determined first combined moisture and the said determined second combined moisture are combined to eliminate the contribution of the added moisture.

2. A method for determining the moisture content of a gas stream according to Claim 1 characterized in that the second quantity of added moisture bears a simple relation to the first added quantity.

3. A method for determining the moisture content of a gas stream according to Claims 1 or 2 characterized in that the added quantities of moisture leads to a moisture content in the range above one part per million.

4. An apparatus for the determination of moisture content in a gas stream comprising a conduit (2) for containing the gas stream a hygrometer (8) means located in the stream and means (6) including an auxiliary passage means (14) whereby first and second quantities of moisture can be added to the gas stream which means is located upstream of the hygrometer means.

**5.** Apparatus for the determination of moisture content according to claim 4 characterized in that the means for introducing first and second quantities of added moisture comprises a diffusion generator.

**6.** Apparatus for the determination of moisture content according to claims 4 or 5 characterized in that the means for introducing a first and second quantity of added moisture comprises a means for providing that a known fraction of the augmented stream shall by-pass the hygrometer means.

**7.** Apparatus for the determination of moisture content in a gas stream according to claims 4, 5 or 6, characterized in that there is provided a main conduit (2) for containing the gas stream, a branch conduit (4) for diverting a fraction of the gas stream, a means for delivering a quantity of moisture (6) in unit time into the gas stream, a hygrometer (8) for measuring the moisture content of the gas stream, flow orifices (10, 16) for determining the rate of flow of the gas stream through the main conduit 2 and the branch conduit 14, a valve (18) for adjusting the volume of the gas steam flowing through the main conduit (2) and the branch conduit (14), and a flow meter (12) for monitoring the rate of flow of the gas stream through the apparatus.

**Patentansprüche**

**1.** Ein Verfahren zur Bestimmung des Feuchtigkeitsgehaltes eines Gasstromes, wobei der Feuchtigkeitsgehalt weniger sein kann als 1 ppm, und worin der Feuchtigkeitsgehalt gleichfalls möglicherweise während der Messung variieren kann, dadurch gekennzeichnet, daß:
(a) eine erste Feuchtigkeitsmenge zu dem Strom hinzugefügt wird und der erste kombinierte Feuchtigkeitsgehalt bestimmt wird;
(b) eine zweite Menge an Feuchtigkeit zu dem Strom hinzugefügt wird, die ein vorherbestimmtes Verhältnis an der ersten Menge aufweist, und der zweite kombinierte Flüssigkeitsgehalt bestimmt wird;
(c) die Ergebnisse der bestimmten ersten kombinierten Feuchtigkeit und der bestimmten zweiten kombinierten Feuchtigkeit kombiniert werden, um den Beitrag der hinzugefügten Feuchtigkeit zu eliminieren.

**2.** Ein Verfahren zur Bestimmung des Feuchtigkeitsgehaltes eines Gasstromes nach Anspruch 1, dadurch gekennzeichnet, daß die zweite Menge der hinzugefügten Feuchtigkeit ein einfaches Verhältnis zu der ersten hinzugefügten Menge aufweist.

**3.** Ein Verfahren zur Bestimmung des Feuchtigkeitsgehaltes eines Gasstromes nach den Ansprüchen 1 oder 2, dadurch gekennzeichnet, daß die hinzugefügten Mengen der Feuchtigkeit zu einem Feuchtigkeitsgehalt in dem Bereich von oberhalb einem Teil pro Million führen.

**4.** Eine Vorrichtung zur Bestimmung des Feuchtigkeitsgehaltes in einem Gasstrom, die eine Leitung (2) zum Enthalten des Gasstromes umfaßt, eine Hygrometervorrichtung (8), die in dem Strom angeordnet ist, sowie eine Vorrichtung (6), welche eine Hilfspassagenvorrichtung (14) enthält, wobei erste und zweite Mengen von Feuchtigkeit zu dem Gasstrom hinzugefügt werden können, wobei die Vorrichtung stromaufwärts von der Hygrometervorrichtung angeordnet ist.

**5.** Vorrichtung zur Bestimmung des Feuchtigkeitsgehaltes gemäß Anspruch 4, dadurch gekennzeichnet, daß die Vorrichtung zum Einführen der ersten und zweiten Menge der hinzugefügten Feuchtigkeit einen Diffusionsgenerator umfaßt.

**6.** Vorrichtung zur Bestimmung des Feuchtigkeitsgehaltes gemäß den Ansprüchen 4 oder 5, dadurch gekennzeichnet, daß die Vorrichtung zum Einführen einer ersten und zweiten Menge der hinzugefügten Feuchtigkeit eine Vorrichtung umfaßt, die dafür sorgt, daß ein bekannter Bruchteil des vergrößerten Stromes um die Hygrometervorrichtung herumgeführt werden soll.

**7.** Vorrichtung zur Bestimmung des Feuchtigkeitsgehaltes in einem Gasstrom gemäß den Ansprüchen 4, 5 oder 6, dadurch gekennzeichnet, daß eine Hauptleitung (2) bereitgestellt wird, um den Gasstrom zu enthalten, ein Nebenzweig (4), um einen Teil des Gasstomes umzuleiten, eine Vorrichtung (6) zum Übergeben einer Feuchtigkeitsmenge pro Einheitszeit in den Gasstrom, ein Hygrometer (8) zum Messen des Feuchtigkeitsgehaltes des Gasstromes, Flußöffnungen (10, 16) zum Bestimmen der Flußrate des Gasstromes durch die Hauptleitung (2) und den Nebenzweig (14), ein Ventil (18) zum Justieren des Volumens des Gasstromes, der durch die Hauptleitung (2) und die Nebenleitung (14) fließt, und ein Strömungsmesser (12) zum Überwachen der Flußrate des Gasstromes durch die Vorrichtung.

## Revendications

1. Méthode de détermination du degré d'humidité d'un courant de gaz, comprenant les phases de:

   (a) ajouter une première quantité d'humidité et déterminer le premier degré d'eau combinée;

   (b) ajouter au courant une seconde quantité d'eau ayant une relation déterminée avec ladite quantité ajoutée et déterminer le second degré d'humidité combinée; et

   (c) combiner les résultats obtenus en (a) et (b) ci-dessus pour éliminer la contribution de l'humidité ajoutée.

2. Méthode de détermination du degré d'humidité dans un courant de gaz suivant la revendication 1, caractérisé en ce que la seconde quantité d'humidité est en relation simple avec la première quantité ajoutée.

3. Méthode de détermination du degré d'humidité dans un courant de gaz suivant l'une des revendications 1 ou 2, caractérisée en ce que les quantités d'humidité ajoutées conduisent à un degré d'humidité de l'ordre de plus d'un millionième.

4. Appareil de détermination du degré d'humidité dans un courant de gaz, comprenant un conduit (2) pour faire passer le courant de gaz, un moyen hygrométrique (8) situé dans le courant et un moyen (6) comprenant un moyen de passage auxiliaire (14) pour introduire la première et la seconde quantité d'humidité ajoutée au courant, ce moyen étant situé en amont du moyen hygrométrique.

5. Appareil de détermination de degré d'humidité suivant la revendication 4, caractérisé en ce que le moyen d'introduction des première et seconde quantités d'huimidité ajoutée comprend un générateur de diffusion.

6. Appareil de détermination de degré d'humidité suivant la revendication 4 ou 5, caractérisé en ce que le moyen d'introduction d'une première et d'une seconde quantité d'humidité ajoutée comprend un moyen de prévoir qu'une fraction connue du courant accru contournera le moyen hygrométrique.

7. Appareil de détermination du degré d'humidité dans un courant de gaz suivant l'une des revendication 4, 5 ou 6, caractérisé en ce qu'il est prévu un conduit principal (2) pour contenir le courant de gaz, une conduit auxiliaire (14) pour dériver une fraction du courant de gaz, un moyen pour fournir une quantité d'humidité (6) par unité de temps au courant de gaz, un hygromètre (8) de mesure du degré d'humidité du courant de gaz, des orifices (10, 16) pour déterminer le débit du courant de gaz dans le conduit principal (2) et le conduit auxiliaire (14), une vanne (18) pour régler les volumes du courant de gaz dans le conduit principal (2) et le conduit auxiliaire (14), et un débitmètre (12) de commande du débit de courant de gaz dans l'appareil.

Sample Gas

DG

S

A

B V

FM